# EUROPEAN PATENT APPLICATION

(11) **EP 0 600 754 A2**
(43) Date of publication of application: **08.06.1994**
(21) Application number: 93309734.7
(22) Date of filing: 03.12.1993
(51) Int. Cl.: A61M 5/155

(54) **Constant pharmaceutical infuser**

(30) Priority: 03.12.1992 JP 89736/92; 14.06.1993 JP 37073/93
(71) Applicant: Kato Hatsujo Kaisha Ltd., Yokohama-shi Kanagawa-ken (JP)
(72) Inventor: Fujiwara, Masatoshi, c/o Kato Hatsujo Kaisya, Ltd., Yokohama-shi, Kanagawa-ken (JP); Asano, Hiroyuki, c/o Kato Hatsujo Kaisya, Ltd., Yokohama-shi, Kanagawa-ken (JP); Okata, Ichizo, c/o Kato Hatsujo Kaisya, Ltd., Yokohama-shi, Kanagawa-ken (JP); Kato, Tsutomu, c/o Kato Hatsujo Kaisya, Ltd., Yokohama-shi, Kanagawa-ken (JP)
(74) Representative: Piesold, Alexander J.

(57) **Abstract**

A constant pharmaceutical infuser comprising a casing 12 having therein an airtight chamber 14 and a gas cylinder holding chamber 15; a pharmaceutical infusion solution filling container 18 capable of undergoing a decrease in internal volume upon application of pressure, provided in the airtight chamber 14 and having an outlet 19 that communicates with the outside of the casing and to which a hypodermic needle 23 is connected through a tube 21 and a flow rate control valve 22; a miniature gas cylinder held in the gas cylinder holding chamber 15 and capable of feeding a pressurized gas to the inside of the airtight chamber 14 when unsealed; and a pressure control valve 28 provided at a gas feed opening of the miniature gas cylinder 24, and capable of closing the gas feed opening when the gas pressure inside the airtight chamber 14 becomes higher than a given value and opening the gas feed opening when the gas pressure inside the airtight chamber 14 becomes lower than the given value. The gas in the miniature gas cylinder 24 flows into the airtight chamber 14 through the pressure control valve 28, whereupon the pharmaceutical infusion solution enclosed in the pharmaceutical infusion solution filling container 18 is forced out at a constant flow rate by the action of the gas pressure and is administered to a patient through the tube 21, the flow rate control valve 22 and the hypodermic needle 23.

## Description

This invention relates to a constant pharmaceutical infuser which may be used to constantly administer pharmaceutical infusion solutions to human bodies. More particularly, it relates to a constant pharmaceutical infuser that is portable and can be produced at a relatively low cost.

In administration of pharmaceutical infusion solutions used for treating adult-disease patients susceptible to physical exhaustion, it is necessary to provide a pharmaceutical infusion system capable of making administration over a long period of time in accordance with the physical condition of patients. Pharmaceutical infusion solutions used for such administration include solutions of anti-cancer agents, analgesics, local anaesthetics, blood glucose level adjustors and so forth. These pharmaceutical infusion solutions are intravenously, intraarterially or subcutaneously administered to bodies.

In such pharmaceutical infusion systems, devices that administer the pharmaceutical infusion solutions little by little by means of a pump as a drive means have been conventionally used. In such apparatus, however, the noise of the pump driving means may hurt patients' ears, and the apparatus have been unsuitable for certain patients. Such apparatus also tend to be too large to be always portable, and hence have been expensive and inappropriate for use in many cases.

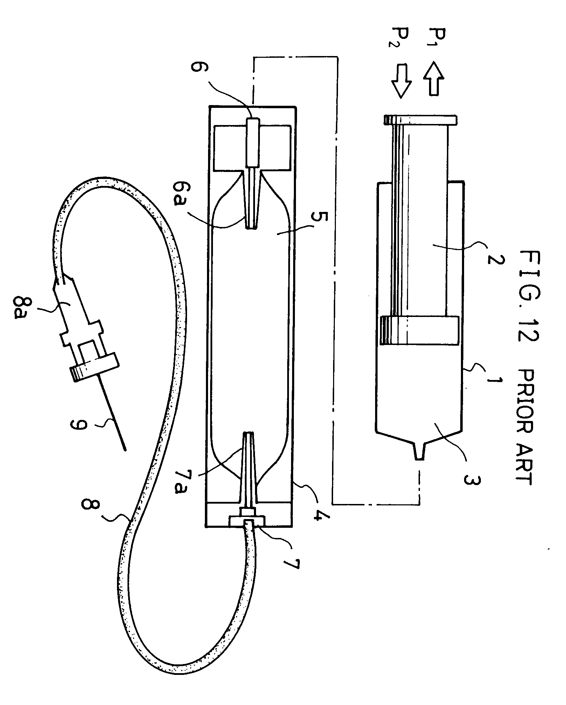

Accordingly, in recent years, pharmaceutical infusion systems making use of no drive means such as pumps have been developed. Fig. 12 shows an example of such a device.

The device shown in Fig. 12 is provided with a cylindrical filling container 4 having a balloon 5 therein. The filling container 4 is fitted at its rear end with a pharmaceutical infusion solution fill opening 6 having a check valve therein. A tubular front end 6a of this fill opening 6 is inserted into the balloon 5. To the front end of the filling container 4, a pharmaceutical infusion solution outlet 7 is fitted, and a tubular rear end 7a of this outlet 7 is inserted into the balloon 5. One end of a tube 8 is also connected to the infusion solution outlet 7, and a hypodermic needle 9 is connected to the other end of the tube through a flow rate control tube 8a.

When using this device, first, a separate hypodermic syringe 1 is provided and its piston 2 drawn toward an arrow P₁ so that a pharmaceutical infusion solution 3 is sucked in. Then, the syringe is put to the infusion solution fill opening 6 provided at the rear end of the filling container 4, where the piston 2 is pushed toward an arrow P₂ so that the infusion solution 3 is injected into the balloon 5 through the fill opening 6. Next, the hypodermic needle 9 is inserted into, for example, a blood vessel of the patient, and then the infusion solution is administered to the human body from the balloon 5 through the infusion solution outlet 7, the tube 8, the flow control tube 8a and the hypodermic needle 9. The flow rate of the solution is adjusted by the action of the flow rate control tube 8a, utilizing the deflating force of the balloon 5.

Such a pharmaceutical infusion system has a simple structure and can be manufactured at a low cost, and hence has the advantages that it is disposable and also is so light-weight that it can be carried.

However, as the device shown in Fig. 12 utilizes the deflating force of the balloon 5 to force out the infusion solution, it is difficult to make the discharge velocity of the infusion solution constant. In particular, when the balloon 5 is almost deflated and the pharmaceutical infusion solution has almost all been delivered, the discharge velocity of the solution abruptly increases, so that a large quantity of the pharmaceutical infusion solution is administered at one time. Hence, when, for example, a device of this type is used to deliver an analgesic such as morphine to a patient suffering from terminal cancer, it follows that the analgesic is administered in a large quantity at one time, possibly endangering the patient's life.

In addition, there is also a possibility of the balloon breaking during the use of the device, and therefore the device has problems of poor safety and reliability.

Moreover, since the balloon expands in a cylindrical form, the device cannot easily be produced in shapes other than cylinders. Thus, for example, it cannot be produced in a shape that makes it easy to put in a pocket, and therefore it tends to be inconvenient to carry.

The present invention was made taking account of such problems involved in the prior art.

According to the invention there is provided a constant pharmaceutical infuser comprising an air-tight chamber connected to a source of gas pressure, an infusion solution container of variable internal volume located in, or formed by the air-tight chamber, and a conduit connected to the infusion solution container, wherein, in use the gas pressure within the air-tight chamber causes the internal volume of the infusion solution container to reduce, thereby forcing a solution out of the infusion solution container.

According to another aspect of the invention there is provided a constant pharmaceutical infuser comprising:
a casing having therein an airtight chamber;
a pharmaceutical infusion solution filling container capable of undergoing a decrease in internal volume upon application of pressure, provided in the airtight chamber of the casing and having an outlet that communicates with the outside of the casing;
a miniature gas cylinder held in the casing and capable of feeding a pressurized gas to the inside of the airtight chamber when unsealed;
a pressure control valve provided at a gas feed opening of the miniature gas cylinder, and capable of closing the gas feed opening of the miniature gas cylinder when the gas pressure inside the airtight chamber becomes higher than a given value and opening the gas feed opening when the gas pressure inside the airtight chamber becomes lower than the given value; and
a hypodermic needle connected to the outlet of the pharmaceutical infusion solution filling container through a tube and a flow-rate control valve.

Preferably, the airtight chamber is provided with a safety valve capable of allowing gas to escape when the gas pressure inside the airtight chamber becomes higher than a given value.

The pharmaceutical infusion solution filling container may preferably be formed of a flexible container made of, for example, plastic or alternatively comprise a container having a cylinder and a piston inserted into the cylinder.

The casing may preferably be provided with a pressure release mechanism capable of releasing the gas in the airtight chamber to the outside.

In the pharmaceutical infusion solution filling container, a fill opening through which the container is filled with the pharmaceutical infusion solution from outside may preferably be provided, and the fill opening may preferably be provided therein with a check valve.

An embodiment of the invention will now be described by way of example only and with reference to the accompanying drawings, in which:-

Fig. 1 is a partially cut away perspective illustration of an embodiment of the constant pharmaceutical infuser according to the present invention.

Figs. 2A and 2B are cross sections to illustrate examples of the check valve provided at the pharmaceutical infusion solution fill opening of the infuser.

Fig. 3 is a partial cross section to illustrate the structure of a gas cylinder holding chamber of the constant pharmaceutical infuser.

Fig. 4 is a partial cross section to illustrate the structure of a pressure control valve and a safety valve of the constant pharmaceutical infuser.

Figs. 5A and 5B are each a partial cross section to illustrate the structure of a sealing portion of the pressure control valve of the constant pharmaceutical infuser.

Fig. 6 is a partial cross section to illustrate a pressure release mechanism of the constant pharmaceutical infuser.

Fig. 7 is a cross section to illustrate another embodiment of the constant pharmaceutical infuser according to the present invention.

Fig. 8 is a partial cross section to illustrate the structure of a pressure control valve of the constant pharmaceutical infuser.

Fig. 9 is a partially cutaway perspective illustration of still another embodiment of the constant pharmaceutical infuser according to the present invention.

Fig. 10 is a cross section to illustrate a further embodiment of the constant pharmaceutical infuser according to the present invention.

Fig. 11 is a cross section to illustrate a still further embodiment of the constant pharmaceutical infuser according to the present invention.

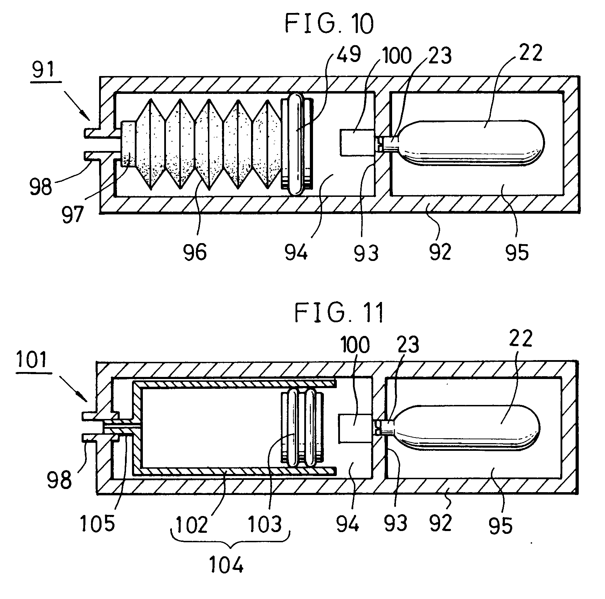

Fig. 11 is a diagrammatic cross section to illustrate a conventional constant pharmaceutical infuser.

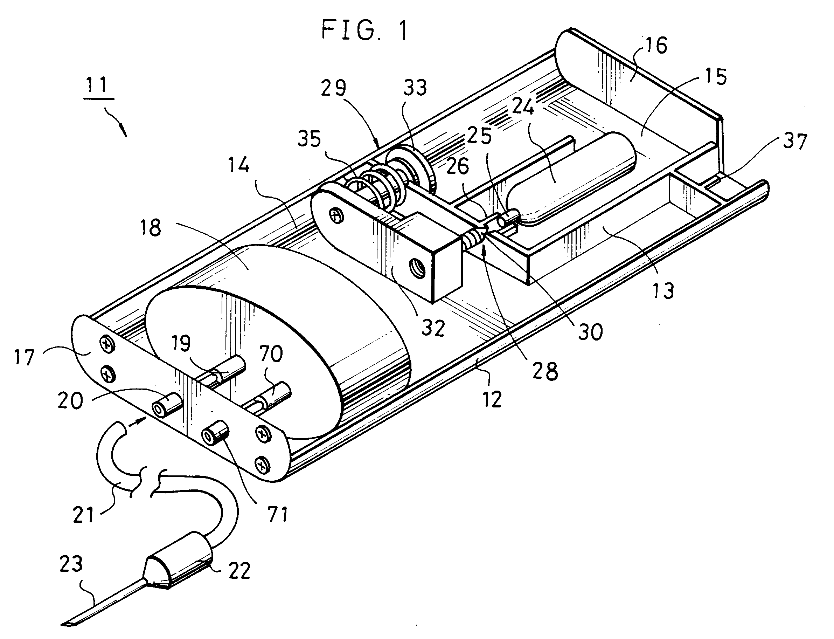

Fig. 1 illustrates an embodiment of the constant pharmaceutical infuser according to the present invention.

This constant pharmaceutical infuser, denoted by reference numeral 11, comprises a casing 12 made of plastic and is molded in the form that can fit the body when carried by patient's side. This casing is provided therein with an airtight chamber 14 and a gas cylinder holding chamber 15 which are divided into them by a partition wall 13. One end of the casing 12 is formed of a rear cover 16 that can be right and left slidably opened and shut, and the other end of the casing 12 is formed of a front cover 17 that is detachably fitted through means of screws or the like. The casing 12 is openable at its top surface, and has a top cover (not shown) by which the casing is closed at its top surface. Both the body and the top cover of the casing are put together so that the airtight chamber 14 can be sealed.

In the airtight chamber 14, a pharmaceutical infusion solution filling container 18 is received, which comprises a container made of plastic, having a flexibility, and is filled with a required pharmaceutical infusion solution in a sterilized state. This container 18 has a pharmaceutical infusion solution outlet 19 connected to a pipe 20 that communicates with the outside of the casing 12. A cover or valve capable of temporarily preventing the pharmaceutical infusion solution from flowing may be provided anywhere through the solution outlet 19 and the pipe 20. The pharmaceutical infusion solution filling container 18 is also provided with a fill opening 70 through which the container is filled with a pharmaceutical infusion solution from the outside. This fill opening 70 is connected to a pipe 71 that communicates with the outside of the casing, and a check valve is provided in the pipe 71.

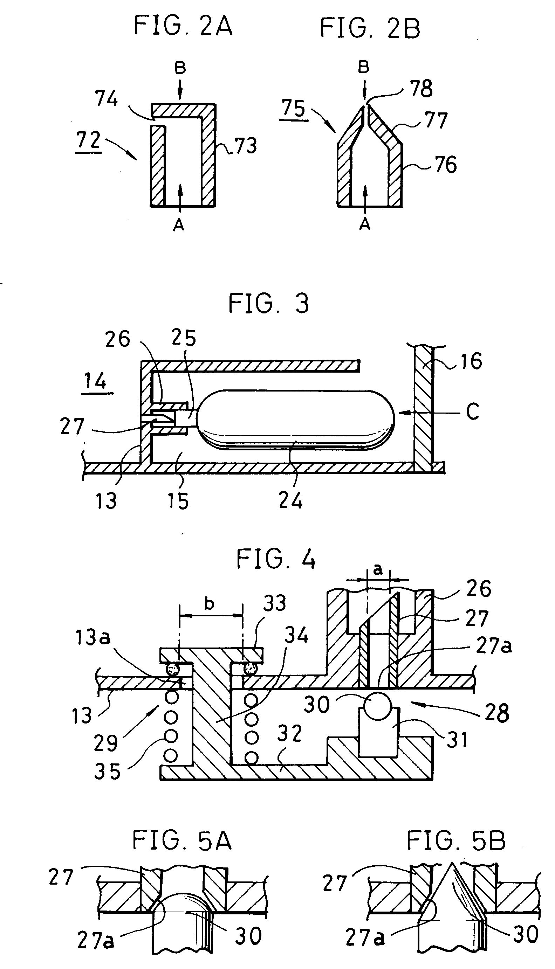

Figs. 2A and 2B illustrates examples of the check valve. A check valve 72 shown in Fig. 2A comprises a cylindrical member 73 whose one end is closed and in which a cutout 74 is made. The valve is so designed that, when a pharmaceutical infusion solution flows in the direction of an arrow A shown in the drawing, the cutout 74 is kept open, and when the pharmaceutical infusion solution happens to flow in the direction of an arrow B shown in the drawing, the cutout 74 is shut. A check valve 75 shown in Fig. 2B comprises a cylindrical member 76 whose one end is closed with tapers and at the center of which a cutout 78 is made. The valve is so designed that, when a pharmaceutical infusion solution flows in the direction of an arrow A shown in the drawing, the cutout 78 is kept open, and when the pharmaceutical infusion solution happens to flow in the direction of an arrow B shown in the drawing, the cutout 78 is shut.

To the pipe 20 that communicates with the outside of the casing 12, a flexible tube 21 with a suitable length is connected, and a hypodermic needle 23 is fitted to the front end of the tube 21 through a flow rate control valve 22. The flow rate control valve 22 may be selected from those capable of achieving a suitable flow rate with respect to a pressure applied from the miniature gas cylinder described below.

A miniature gas cylinder 24 comprising a metal cylindrical capsule filled with, for example, carbonic acid gas, nitrogen gas or air is held in the gas cylinder holding chamber 15. The miniature gas cylinder 24 may preferably be filled with the gas at a gas pressure of from 5 to 8 kg/cm². In the present embodiment, a gas cylinder in which 0.05 g of nitrogen gas has been enclosed at a pressure of 7.5 kg/cm² (available from Nihon Tansan Gasu K.K.) is used.

With reference also to Fig. 3, this miniature gas cylinder 24 is inserted at its mouth 25 into a pipe 26 formed on the partition wall 13 of the casing 12, and a hollow needle 27 for communicating with the gas cylinder through the partition wall 13 is inserted into the pipe 26. The casing 12 also has the rear cover 16 on the rear side of the miniature gas cylinder 24 , which is designed as follows: The rear cover can be slided to make it open and then the rear side of the miniature gas cylinder 24 can be pushed with a finger, so that the hollow needle 27 can stick or run into the mouth 25 to unseal the gas cylinder and the nitrogen gas enclosed in the gas cylinder 24 can flow into the airtight chamber 14 through the hollow needle 27. A screw or a push button may be provided at the part corresponding to the rear cover 16 so that the miniature gas cylinder 24 can be pushed forward by operating the screw or the push button while the rear cover 16 is kept close.

Once the miniature gas cylinder 24 has been unsealed by pushing forward it in this way, the nitrogen gas in the gas cylinder 24 flows into the airtight chamber 14 through the hollow needle 27. If no valve is provided at this flow path, the gas flow may stop at the time when the gass pressure in the airtight chamber 14 becomes equal to the gas pressure in the gas cylinder 24. Since in such an instance the gas pressure becomes reasonably high, this is not preferable from the viewpoint of pressure resistance or the like of the casing 12. If only a safety valve is provided in the airtight chamber 14 of the casing 12, the gas can escape through the safety valve when the gas pressure becomes higher than a given value as a result of the flowing of gas into the airtight chamber 14, but the gas may continue to flow out until the gas pressure in the miniature gas cylinder 24 becomes lower than the gas pressure set by the safety valve, bringing about the problem that the gas inside the miniature gas cylinder 24 is soon used up.

To eliminate such a problem, the constant pharmaceutical infuser 11 of the present embodiment is provided with a pressure control valve 28 capable of closing the gas feed opening of the miniature gas cylinder 24 when the gas pressure inside the airtight chamber 14 becomes higher than a given value and opening the gas feed opening when the gas pressure becomes lower than the given value, and a safety valve 29 capable of leaking the gas from the airtight chamber 14 to the outside when the gas pressure inside the airtight chamber 14 becomes higher than the given value.

With reference also to Fig. 4, the pressure control valve 28 and the safety valve 29 are integrally formed on a supporting plate 32 provided in the airtight chamber 14, and are so designed as to be opened and closed interlockingly with each other. More specifically, in the partition wall 13, a gas feed opening 27a corresponding to the opening of the hollow needle 27 which is stuck into the mouth 25 of the miniature gas cylinder 24 and a gas outlet 13a through which the gas inside the airtight chamber 14 is leaked are formed.

The pressure control valve 28 is provided projectingly from the supporting plate 32 and is comprised of a shaft 31 extending toward the gas feed opening 27a and a valve element 30 provided on the top of the shaft 31 and capable of opening and closing the gas feed opening 27a. As the valve element 30, it is possible to use an element having a round tip as shown in Fig. 5A or an element having a needlelike tip as shown in Fig. 5B.

As for the safety valve 29, it is comprised of a valve element 33 so shaped as to stop up the gas outlet 13a in the partition wall 13 from the outside of the airtight chamber 14, a shaft 34 extending from this valve element 33 in the shape of a handle to come inside the airtight chamber 14 and connected at its another end with the supporting plate 32 in the airtight chamber 14, and a spring 35 fitted around the shaft, pressed at its lower end (as viewed in Fig. 4) against the supporting plate 32 and pressed at its upper end (as viewed in Fig. 4) against the partition wall 13 on the airtight chamber 14 side.

Now, assume that the gas feed opening 27a has an inner diameter a of 2 mm, the gas outlet 13a has an inner diameter b of 12.5 mm and the miniature gas cylinder 24 is filled with gas at a gas pressure of 5 kg/cm², the load applied to the valve element 30 at the gas feed opening 27a is 160 g. Meanwhile, assume that the inside of the airtight chamber 14 is set at a pressure of 0.4 kg/cm², the load applied to the valve element 33 at the gas outlet 13a is 490 g. Since, however, the latter's load decreases on account of the force of the spring 35 pressing against the valve element, the load applied to the valve element 30 from the outside of the airtight chamber 14 and the load applied to the valve element 33 from the inside of the airtight chamber 14 can be balanced when the spring force of the spring 35 is set at 330 g.

More specifically, when the pressure inside the airtight chamber 14 is lower than 0.4 kg/cm², the load applied to the valve element 30 from the outside of the airtight chamber 14 exceeds the other load, so that the valve element 30 is opened and the gas flows into the airtight chamber 14. At this time, the valve element 33 is kept close since the supporting plate 32 is pressed inwards. When the pressure inside the airtight chamber 14 becomes higher than 0.4 kg/cm², the load applied to the valve element 33 from the inside of the airtight chamber 14 exceeds the other load, so that the valve element 33 is opened and the gas flows out of the airtight chamber 14. At this time, the valve element 30 is kept closed since the supporting plate 32 is pressed outwards. Thus, the pressure inside the airtight chamber 14 can be always kept at around 0.4 kg/cm², so that the pharmaceutical infusion solution held in the pharmaceutical infusion solution filling container 18 can be forced out at a constant flow rate.

In the present invention, only the pressure control valve 28 may be provided without providing the safety valve 29.

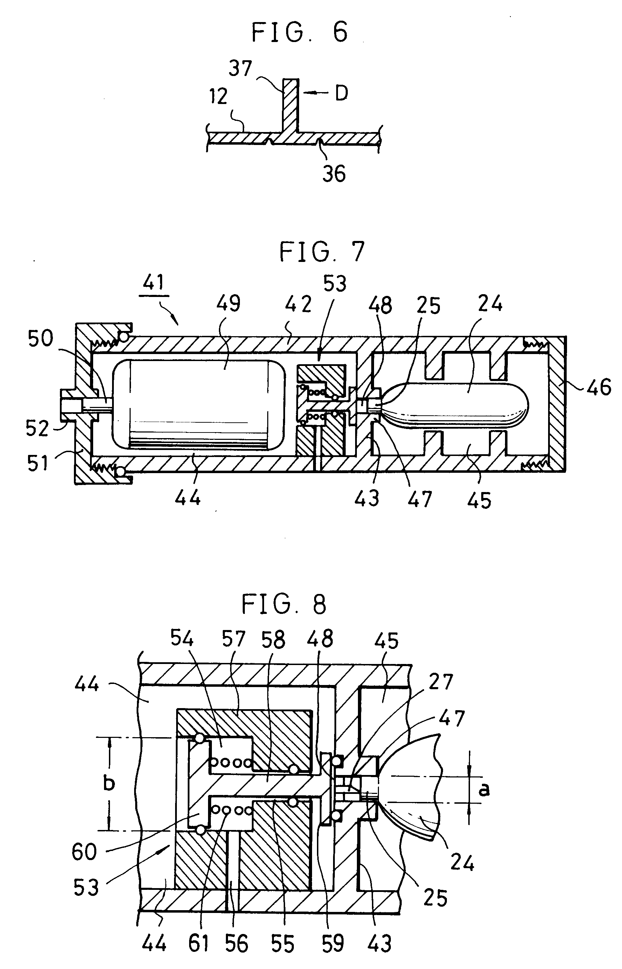

In the present constant pharmaceutical infuser 11, a pressure release mechanism capable of discharging the gas remaining in the miniature gas cylinder 24 before its disposal is provided taking account of instances in which the infuser 11 having been used is disposed. With reference also to Fig. 6, this pressure release mechanism is comprised of the wall of the casing 12 in any desired portion of which, for example, a circular groove 36 along which the wall has a small thickness is formed and a projection 37 is formed outwards at this portion. Thus, when the projection 37 is strongly pushed in the direction as shown by an arrow D in the drawing, the wall of the casing is broken along the portion 36 with a small wall thickness and a hole is made in the wall, so that the gas flowing into the airtight chamber 14 can be released outside. The pressure release mechanism provided in this way enables safe disposal of containers having been used.

Figs. 7 and 8 illustrate another embodiment of the constant pharmaceutical infuser according to the present invention.

This constant pharmaceutical infuser, denoted by reference numeral 41, has a cylindrical casing 42 made of plastic, and the casing 42 is divided into an airtight chamber 44 and a gas cylinder holding chamber 45 by a partition wall 43. At the rear end of the casing 42, a rear cap 46 is detachably secured by a threaded closure means. In the gas cylinder holding chamber 45, the miniature gas cylinder 24 is provided in such a manner: that its mouth 25 is inserted into the pipe 47. In this pipe 47, a hollow needle 27 is provided as shown in Fig. 8, so as to unseal the mouth 25 of the miniature gas cylinder 24 to feed the gas into the airtight chamber 44. The opening of the pipe 47 on the side of the airtight chamber 44 serves as a gas feed opening 48.

In the airtight chamber 44, a pharmaceutical infusion solution filling container 49 comprising a flexible plastic container is provided. A pharmaceutical infusion solution outlet 50 of this container 49 is inserted into the pipe 52 formed through a front cap 51 secured to the front end of the casing 42 by a threaded closure means. To the pipe 52, a tube, a flow rate control valve and a hypodermic needle (which are not shown) are connected in the same manner as the embodiment firstly described.

A pressure control valve 53 is also fitted to the gas feed opening 48 on its inner side of the airtight chamber 44. This pressure control valve 53 is fixed on the inner wall of the casing 44, and has a cylindrical block 57 provided with a recess 54 that opens to the front end side, a through-hole 55 made at the bottom of the recess toward the rear end side, and a vent hole 56 through which the inside of the recess 54 is made open to the outside. This block 57 is fitted with a sliding member having a shaft 58 inserted into the through-hole 55, a valve element 59 fixed on one end of this shaft and capable of opening and closing the gas feed opening 48, and a piston 60 fixed on the other end of the shaft 58 and capable of slidably moving inside the recess 54. A spring 61 is fitted between the piston of this sliding member and the bottom wall of the recess 54 so that the sliding member is pressed against the front end side of the casing 44.

Now, assume that the gas feed opening 48 has an inner diameter a of 2 mm, the recess 54 has an inner diameter b of 12.5 mm and the miniature gas cylinder 24 is filled with gas at a gas pressure of 5 kg/cm², the load applied to the valve element 59 at the gas feed opening 48 is 160 g. Meanwhile, assume that the inside of the airtight chamber 44 is set at a pressure of 0.4 kg/cm², the load that acts to push the piston 60 at the opening of the recess 54 is 490 g. Since, however, the latter's load decreases on account of the force of the spring 35 pressing against it, the load that acts to push the valve element 59 toward the inside of the airtight chamber 44 by the action of the gas pressure in the miniature gas cylinder 24 and the load that acts to push the piston 60 toward the outside of the airtight chamber 44 can be balanced when the spring force of the spring 35 is set at 330 g.

More specifically, when the pressure inside the airtight chamber 44 is lower than 0.4 kg/cm², the load that acts to push the valve element 59 toward the inside of the airtight chamber 44 exceeds the other load, so that the valve element 59 is opened and the gas flows into the airtight chamber 44. When the pressure inside the airtight chamber 44 becomes higher than 0.4 kg/cm², the load that acts to push the piston 60 toward the outside of the airtight chamber 44 exceeds the other load, so that the valve element 59 is closed and the gas stops flowing. Thus, the pressure inside the airtight chamber 44 can be kept constant, so that the pharmaceutical infusion solution enclosed in the pharmaceutical infusion solution filling container 49 can be forced out at an always constant flow rate.

As a component omitted from illustration in the drawing, the airtight chamber 44 may preferably be provided in its inside with a safety valve in addition to the pressure control valve 53. This safety valve may preferably be set at a pressure a little higher than the pressure set for the pressure control valve 53.

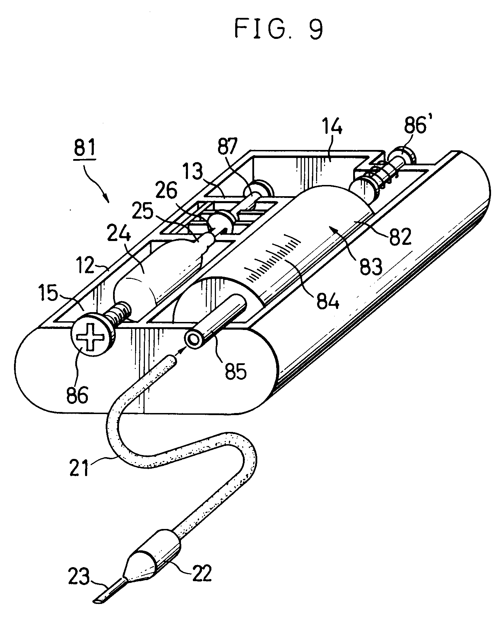

Fig. 9 illustrates still another embodiment of the constant pharmaceutical infuser according to the present invention.

The constant pharmaceutical infuser, denoted by reference numeral 81, has a casing 12 made of plastic, having a flat shape as a whole, in the same way as the embodiment shown in Fig. 1. This casing 12 is provided therein with an airtight chamber 14 and a gas cylinder holding chamber 15 which are divided into them by a partition wall 13. The casing 12 is openable at its top surface, and has a top cover (not shown) by which the casing is closed at its top surface. Both the body and the top cover of the casing are put together so that the airtight chamber 14 can be sealed.

In the airtight chamber 14, a pharmaceutical infusion solution filling container 83 comprised of a cylinder 82 and a piston (not shown) provided in its inside are received. Scale 84 is marked on the cylinder 82 so that the quantity of the infusion solution remaining therein can be ascertained according to the position of the piston in its inside. Accordingly, the casing 12 is set transparent at its part corresponding to the scale 84 so that the scale can be seen from the outside. On one end of the cylinder 62, a pharmaceutical infusion solution outlet 85 is formed, and this outlet 85 extends outwards to communicate with the outside of the casing 12. To this outlet 85, a flexible tube 21 with a suitable length is connected, and a hypodermic needle 23 is fitted to the front end of the tube 21 through a flow rate control valve 22.

The same miniature gas cylinder 24 as that in the embodiments previously described is held in the gas cylinder holding chamber 15. This miniature gas cylinder 24 is inserted at its mouth 25 into a pipe 26 formed on the partition wall 13 of the casing 12, and a hollow needle (not shown) for communicating with the gas cylinder through the partition wall 13 is inserted into the pipe 26. Once a set screw 86 fitted to the casing 12 is turned forward to push the rear side of the miniature gas cylinder 24, the hollow needle can stick or run into the mouth 25 to unseal the gas cylinder and the nitrogen gas enclosed in the gas cylinder 24 can flow into the airtight chamber 14 through the hollow needle. A pressure control valve 87 is fitted to the gas feed opening of the pipe 26.

The casing 12 is also provided with a safety valve 86' capable of leaking the gas from the airtight chamber 14 to the outside when the gas pressure inside the airtight chamber 14 becomes higher than a given value.

In this constant pharmaceutical infuser 81, a piston (not shown) of the pharmaceutical infusion solution filling container 83 is pushed when the gas fed from the miniature gas cylinder 24 makes the gas pressure in the airtight chamber 14 higher, so that the pharmaceutical infusion solution enclosed in its filling container 18 is forced out and administered to a patient through the tube 21, the flow rate control valve 22 and the hypodermic needle 23. The patient may watch the scale 84 to ascertain the position of the piston seen through the cylinder 82, so that they can learn an accurate quantity of the pharmaceutical infusion solution remaining in its filling container 18.

Fig. 10 illustrates a further embodiment of the constant pharmaceutical infuser according to the present invention.

This constant pharmaceutical infuser, denoted by reference numeral 91, has a cylindrical casing 92 made of plastic. The casing 92 is divided into an airtight chamber 94 and a gas cylinder holding chamber 95 by a partition wall 93.

The airtight chamber 94 is provided therein with a pharmaceutical infusion solution filling container 96 having a bellows structure so as to be expandable or contractible, and a pharmaceutical infusion solution outlet 97 of the container 96 is connected to a pipe 98 that communicates with the outside of the casing 92. To this pipe 98, a tube, a flow rate control valve and a hypodermic needle (which are not shown) are connected in the same fashion as the embodiments previously described. To the rear end of the pharmaceutical infusion solution filling container 97, a piston 99 that can fit the airtight chamber 94 is provided so that the gas flowing into the airtight chamber 94 pushes the filling container 96 by its pressure to force out the pharmaceutical infusion solution.

In the gas cylinder holding chamber 95, a miniature gas cylinder 22 is received, and its mouth 23 is inserted into the airtight chamber 94 so as to communicate with its inside through the partition wall 93. As a component omitted from illustration in the drawing, an unsealing member comprising a hollow needle or the like is also provided in the vicinity of the mouth 23 in the same fashion as in the embodiments previously described. A pressure control valve 100 is also fitted to an opening at which gas is fed to the airtight chamber 94 through the mouth 23.

Thus, once the hollow needle (not shown) is run into the mouth of the miniature gas cylinder 22, the gas flows into the airtight chamber 94 and pushes the piston 99 to contract the bellows type pharmaceutical infusion solution filling container 96, so that the pharmaceutical infusion solution can be made to constantly flow through the tube, the flow rate control valve and the hypodermic needle (which are not shown). As a component omitted from illustration in the drawing, the airtight chamber 94 may preferably be provided with a safety valve in the same manner as the embodiments previously described.

Fig. 11 illustrates a still further embodiment of the constant pharmaceutical infuser according to the present invention. Substantially the same components as those in the embodiment shown in Fig. 10 are denoted by the same reference numerals, and description thereon is omitted here.

In this constant pharmaceutical infuser, denoted by reference numeral 101, a pharmaceutical infusion solution filling container 104 comprised of a cylinder 102 and a piston 103 is used. This pharmaceutical infusion solution filling container 104 is provided in the airtight chamber 94, and its pharmaceutical infusion solution outlet 105 is connected to the pipe 98 that communicates with the outside of the casing 92. Thus, the gas flowing into the airtight chamber 94 from the miniature gas cylinder 22 pushes the the piston 103 by its pressure to force out the pharmaceutical infusion solution from the inside of the cylinder 102.

In the foregoing embodiment, the piston 103 may be directly provided in the airtight chamber 94 so that the airtight chamber 94 itself can be utilized as a cylinder.

In the present invention, the pressure control valve, the safety valve, the pressure release mechanism and so forth are by no means limited to those having the structures described in the embodiments described above, and those having various structures can be employed. The miniature gas cylinder may also be not received completely in the casing, but fitted to the casing in the form of an attachment.

According to the constant pharmaceutical infuser of the present invention, the pharmaceutical infusion solution filling container is filled from the outside in the desired quantity, with a pharmaceutical infusion solution prepared in an appropriate concentration, and is received in the airtight chamber of the casing. Then, once the miniature gas cylinder also received in the casing is unsealed, a high-pressure gas in the miniature gas cylinder flows into the airtight chamber through the pressure control valve and applies a pressure to the pharmaceutical infusion solution filling container provided in the airtight chamber. The pharmaceutical infusion solution filling container has a structure, or is made of a material, that may undergo a decrease in its internal volume, and hence the pharmaceutical infusion solution enclosed in its filling container is forced out to flow out of the hypodermic needle through the tube and the flow rate control valve.

The pressure of the gas flowing out of the miniature gas cylinder is more highly precise and more constant than the pressure produced when a balloon deflates. Such a gas cylinder is also filled with a gas having a much larger volume than the volume of the airtight chamber in the constant pharmaceutical infuser of the present invention. Hence, the pressure applied may undergo no change due to any change in volume that may be caused when the gas is fed into the airtight chamber and the pharmaceutical infusion solution filling container gradually becomes smaller. The gas pressure inside the airtight chamber is maintained at an always constant level by the pressure control valve. Thus, an always constant pressure can be applied to the pharmaceutical infusion solution filling container, and hence the infusion solution can be flowed out at a constant flow rate to the last.

In addition, since the miniature gas cylinder has a more uniform quality than balloons, may cause no deterioration of materials and can not break even when any impact is externally applied more or less, the device can achieve a high safety and a high reliability.

Moreover, since the casing having the airtight chamber is provided therein also with the pharmaceutical infusion solution filling container and the miniature gas cylinder, the degree of freedom in shape can be enhanced. Hence, the device can be made into such a shape, for example, that it is easy to put in a pocket or to belt to carry on, and can be made portable with ease.

In the preferred embodiments of the present invention, the airtight chamber is provided with a safety valve capable of leaking the gas when the gas pressure inside the airtight chamber becomes higher than a given value. This eliminates the possibility that the pharmaceutical infusion solution is administered in a large quantity because of an abnormal high gas pressure in the airtight chamber.

The pharmaceutical infusion solution filling container may also be formed of a flexible container made of plastic or a container comprised of a cylinder and a piston inserted into the cylinder.

The casing may also be provided with a pressure release mechanism capable of releasing the gas in the airtight chamber to the outside. This pressure release mechanism is useful for enabling safe disposal after release of pressure, especially when the casing is used as a disposable container.

In preferred embodiments, the pharmaceutical infusion solution filling container has a fill opening through which the container is filled with the pharmaceutical infusion solution from its outside and the fill opening is provided therein with a check valve. This enables supply of the pharmaceutical infusion solution form the outside when the infusion solution in the filling container becomes short.

Thus it will be seen that, at least in the preferred embodiments there is provided a constant pharmaceutical infuser that can deliver the pharmaceutical infusion solution at a constant rate, provides a high level of safety and allows a significant degree of freedom in the choice of its shape.

## Claims

1. A constant pharmaceutical infuser comprising;
a casing having therein an airtight chamber;
a pharmaceutical infusion solution filling container capable of undergoing a decrease in internal volume upon application of pressure, provided in the airtight chamber of the casing and having an outlet that communicates with the outside of the casing;
a miniature gas cylinder held in the casing and capable of feeding a pressurized gas to the inside of the airtight chamber when unsealed;
a pressure control valve provided at a gas feed opening of the miniature gas cylinder, and capable of closing the gas feed opening of the miniature gas cylinder when the gas pressure inside the airtight chamber becomes higher than a given value and opening the gas feed opening when the gas pressure inside the airtight chamber becomes lower than the given value; and
a hypodermic needle connected to the outlet of the pharmaceutical infusion solution filling container through a tube and a flow-rate control valve.

2. The constant pharmaceutical infuser according to claim 1, wherein said airtight chamber is provided with a safety valve capable of leaking the gas when the gas pressure inside the airtight chamber becomes higher than the given value.

3. The constant pharmaceutical infuser according to claim 1 or 2, wherein said pharmaceutical infusion solution filling container comprises a flexible container made of plastic.

4. The constant pharmaceutical infuser according to claim 1 or 2, wherein said pharmaceutical infusion solution filling container comprises a container comprised of a cylinder and a piston inserted into the cylinder.

5. The constant pharmaceutical infuser according to any preceding claim , wherein said casing is provided with a pressure release mechanism capable of releasing the gas in the airtight chamber to the outside.

6. The constant pharmaceutical infuser according to any preceding claim , wherein said pharmaceutical infusion solution filling container has a fill opening through which the container is filled with the pharmaceutical infusion solution from its outside; said fill opening being provided therein with a check valve.

7. A constant pharmaceutical infuser comprising an air-tight chamber connected to a source of gas pressure, an infusion solution container of variable internal volume located in, or formed by the air-tight chamber, and a conduit connected to the infusion solution container, wherein, in use the gas pressure within the air-tight chamber causes the internal volume of the infusion solution container to reduce, thereby forcing a solution out of the infusion solution container.

8. A constant pharmaceutical infuser as claimed in claim 7, wherein the source of gas pressure comprises a gas cylinder.

9. A constant pharmaceutical infuser as claimed in claim 7 or 8, further comprising a pressure control valve to control the flow of gas from the source of gas pressure to the air-tight chamber and thereby regulate the pressure within the air-tight chamber.

10. A constant pharmaceutical infuser as claimed in claim 7, 8 or 9, further comprising a valve for releasing gas from the air-tight chamber when the pressure within the chamber exceeds a pre-set valve.
